# DEMANDE DE BREVET EUROPEEN

(11) **EP 0 603 061 A1**
(43) Date de publication de la demande: **22.06.1994**
(21) Numéro de dépôt: 93403022.2
(22) Date de dépôt: 14.12.1993
(51) Int. Cl.: G01N 33/68, G01N 33/543, G01N 33/58, C12Q 1/00

(54) **Test de pyrogénicité et kit de mise en oeuvre**

(30) Priorité: 15.12.1992 FR 9215095
(71) Demandeur: MEDGENIX DIAGNOSTICS, B-6220 Fleurus (BE)
(72) Inventeur: De Groote, Donat, B-1410 Waterloo (BE); Franchimont, Paul, B-5280 Modave (BE); Hoffmann, Pierre, B-1060 Bruxelles (BE); Lopez Y Cadenas, Miguel, B-4020 Liège (BE); Vrindts-Gevaert, Yvonne, B-4160 Anthisnes (BE); Meuleman-Gathy,Renée, B-4059 Poulseur (BE)
(74) Mandataire: Ahner, Francis

(57) **Abrégé**

La présente invention a pour objet un test de pyrogénicité consistant dans la détection d'agent(s) pyrogène(s) dans une solution à usage médical ou biologique, dans lequel :
1. On mesure la quantité de substance induite dans la dite solution avant et après culture dans la dite solution de cellules ne produisant des dites substances induites qu'en présence du(des) dit(s) agent(s) pyrogène(s) ou de cellules dont la production de la dite subtance induite est accrue en présence du(des) dit(s) agent(s) pyrogène(s).
2. On évalue la quantité d'agents pyrogènes dans la dite solution par rapport à la différence de quantité de la dite substance induite après et avant la dite culture à l'aide d'une courbe de corrélation entre la quantité d'agent(s) pyrogène(s) et la quantité de la dite substance induite, courbe réalisée en mesurant, dans les mêmes conditions, la quantité de la dite substance induite produite dans des échantillons de solutions initialement dépourvues de la dite substance induite, calibrées en agent(s) pyrogène(s) et cultivées dans les mêmes conditions.

La présente invention a également pour objet un format technologique ou kit utile dans le test de pyrogénicité selon l'invention.

## Description

La présente invention concerne un test de pyrogénicité consistant dans la détection d'agent(s) pyrogène(s) tel(s) que les endotoxines, en particulier les lipopolysaccharides (LPS), dans une solution à usage médical ou biologique.

La détection de la présence d'agents pyrogènes comme les endotoxines est une étape très importante du contrôle de qualité, dans l'industrie pharmaceutique, de toute une série de produits à usage médical ou pour une utilisation en analyse ou en recherche biologique dont par exemple
- toutes les solutions pharmaceutiques injectables, en particulier les solutions utilisées dans les reins artificiels,
- les milieux de culture servant à la recherche biologique ou utilisés dans le cadre d'analyses biologiques.

L'activité pyrogénique est essentiellement due à la présence, dans les solutions concernées, de lipopolysaccharides (LPS), en particulier provenant de la paroi de bactéries gram⁻.

Deux méthodes sont actuellement utilisées pour tester la présence de pyrogènes dans une solution :
- Injection de la solution à tester à des lapins et mesure de l'accroissement de leur température,
- Test au limulus (test LAL).

La première méthode est fastidieuse et consomme beaucoup de temps. La seconde, bien que plus simple et plus rapide, reste néanmoins moyennement reproductible. D'autre part, des fragments de LPS de petits poids moléculaire ne sont pas détectés par ces méthodes.

Une des caractéristiques des endotoxines et notamment des LPS est leur capacité d'induire la production de monokines, en particulier TNFalpha, Il₆, Il₁, Il₈, par les monocytes.

La présente invention fournit un nouveau test de pyrogénicité basé sur la mesure de la production d'une substance induite, en particulier d'une monokine (TNF, Il6 ...) en présence d'agents pyrogènes.

Plus précisément, la présente invention a pour objet un test de pyrogénicité consistant dans la détection d'agent(s) pyrogène(s) tel(s) que les endotoxines, en particulier les lipopolysaccharides (LPS), dans une solution à usage médical ou biologique, dans lequel :
1. On mesure la quantité d'une substance induite telle que des monokines, des prostaglandines, des protéases cytolitiques, l'alpha2-macroglobuline, les ROI (reactive oxygen intermediates), dans la dite solution avant et après culture dans la dite solution de cellules ne produisant des dites substances induites qu'en présence du(des) dit(s) agent(s) pyrogène(s) ou de cellules dont la production de substance induite est accrue en présence du(des) dit(s) agent(s) pyrogène(s), et
2. On évalue la quantité d'agents pyrogènes dans la dite solution par rapport à la différence de quantité de la dite substance induite après et avant la dite culture à l'aide d'une courbe de corrélation entre la quantité d'agent(s) pyrogène(s) et la quantité de substance induite, courbe réalisée en mesurant, dans les mêmes conditions, la quantité de la dite substance induite dans des échantillons de solutions initialement dépourvues de la dite substance induite calibrées en agent(s) pyrogène(s) cultivées dans les mêmes conditions.

De façon tout à fait avantageuse, la présente invention concerne un test de pyrogénicité basé sur un procédé d'évaluation de la production par des cellules vivantes de la dite substance induite, notamment de monokines, dans lequel on effectue une culture cellulaire, et l'on évalue la quantité de la dite substance induite produite par dosage immunologique sur phase solide in vitro.

L'invention a également pour objet un test de pyrogénicité dans lequel le procédé d'évaluation, par méthode immunologique, de la production de substance induite, notamment par des cellules vivantes in vitro, est caractérisé en ce que la culture cellulaire au cours de laquelle s'effectue la production et la phase d'immunocapture du dosage immunologique sont réalisés au sein d'un seul et même complexe récipient-support.

En d'autres termes, le support de la culture et le support de la réaction de dosage immunologique sont tous deux présents ensemble au cours de la phase de production de la molécule à doser, au lieu d'être dissociés dans des phases successives du processus normal d'évaluation.

L'invention concerne également un format technologique conçu et adapté pour ce procédé d'évaluation, notamment tout format comprenant un support à la culture cellulaire dans lequel est déjà intégré un élément du dosage, notamment sous forme d'anticorps fixés sur une phase solide du support ou une phase solide telle que des billes à l'intérieur du support. En dehors de ce support, le format peut comprendre tous milieux ou réactifs utiles à la culture et au dosage in situ sur le site de production en fonction de l'application poursuivie.

Le procédé de dosage in situ, et le format technologique correspondant, qui intègre la culture de la population cellulaire utilisée, et l'immunocaptation nécessaire au dosage en une seule étape au niveau du site de production consiste à cultiver la population cellulaire dans la solution à étudier, par exemple dans les puits d'une plaque de microtitration en présence des éléments nécessaires au dosage d'intérêt. Au moment choisi par l'utilisateur, la culture est interrompue par lavage de la phase solide et le dosage est directement révélé dans la plaque sans transferts du milieu de culture.

Plus précisément, la présente invention a donc pour objet un test de pyrogénicité dans lequel on effectue une culture cellulaire et l'on évalue la quantité de la dite substance induite produite par dosage immunologique sur phase solide in vitro, caractérisé en ce que :
a) la culture cellulaire est effectuée, éventuellement en présence du traceur, dans un récipient qui incorpore la phase solide du dosage sur laquelle sont fixés les éléments de capture spécifiques de la substance induite à doser, et en ce que
b) lorsque le temps de culture est écoulé, après lavage de la phase solide et incubations des éventuels différents autres réactifs nécessaires au dosage, notamment un substrat du traceur en présence de la phase solide, on lit le signal émis par le traceur fixé à la phase solide ou par son dit substrat.

De préférence, dans l'étape b), le traceur et la phase solide sont incubés dans le récipient ayant servi à la culture.

Lorsque le traceur est marqué par un enzyme, les dits réactifs nécessaires au dosage comprennent en effet un substrat de l'enzyme que l'on incube après le dit lavage de la phase solide et on lit alors le signal émis plus précisément par le substrat.

Par "incorporer", on entend dans la présente demande que le support solide du dosage peut être physiquement dépendant du support de la culture (par exemple fond du puits ou du tube de culture) ou physiquement indépendant (par exemple billes ou sphères ajoutées sur le support de culture), l'important étant la présence simultanée des supports de la culture et du dosage.

Par "récipient de culture", on entendra désigner le récipient dans lequel est effectuée la culture, il pourra ou non être la phase solide sur laquelle est effectuée l'immuno-captation.

On a en effet découvert de façon tout à fait surprenante que les éléments et réactifs intervenant dans la culture et les éléments et réactifs nécessaires au dosage :
- d'une part étaient stables et parfaitement compatibles lorsqu'ils sont mis en présence et mis en oeuvre dans le même récipient-support dans les conditions de culture, que ce soit notamment pour les éléments de captures fixes à la phase solide ou pour le traceur, et
- d'autre part, n'interféraient pas négativement sur la qualité de la culture et de la production des monokines à doser.

Dans certains cas, notamment lors d'un dosage de type sandwich, le traceur peut être mis à incuber en présence de la phase solide après-un premier lavage de celle-ci intervenant après la production de la substances induites à doser par culture cellulaire et son immunocaptation. Après incubation du traceur et un deuxième lavage de la phase solide, on effectue la lecture du signal émis après incubation éventuelle avec les autres réactifs nécessaires au dosage.

Selon une variante tout à fait avantageuse du procédé selon l'invention, la phase solide consiste dans la surface intérieure du récipient de culture sur laquelle sont fixés notamment par absorption ou par lien chimique les éléments de capture spécifiques de la ou des monokines à doser.

Selon une autre variante, la phase solide du dosage est constituée par un ou des éléments physiquement indépendants des récipients de culture cellulaire. Par exemple, il peut s'agir de billes revêtues, notamment par absorption ou par lien chimique, desdits éléments de capture. Ladite phase solide pouvant être ajoutée dans ledit récipient soit au début de la culture, soit en cours de culture. Parmi les éléments physiquement indépendants, il faut citer également les éléments solidaires d'une pièce de préhension, notamment un couvercle, qui peuvent ainsi être transférés dans un autre récipient après l'étape de culture.

Dans un mode de réalisation illustrant l'invention, la population cellulaire à utiliser, éventuellement diluée dans un milieu de culture adéquat contenant facultativement des agents activateurs, c'est-à-dire stimulateurs de la production de la dite substance induite, est distribuée quantitativement sur un support de culture au niveau duquel se fera la production de la dite substance induite à mesurer, ce support comprenant d'emblée un élément essentiel du futur dosage sous forme d'anticorps fixés de manière adéquate sur une phase solide qui peut être le support. Au moment choisi, la culture est interrompue par un lavage approprié des cellules et le dosage de la substance induite est directement achevé sur le support sans transfert supplémentaire du milieu de culture.

Ainsi, par exemple, dans un mode de réalisation particulier, on dilue du sang humain complet fraîchement prélevé sur un individu sain, par exemple au 1/5ème ou au 1/10ème, dans un milieu de culture contenant l'endotoxine à doser, et on distribue cette suspension cellulaire sur une plaque de microtitration sur laquelle sont déjà fixés des anticorps nécessaires aux dosages. La plaque est mise à incuber dans des conditions standardisées (température, humidité, pression partielle en O₂ et CO₂, etc...) pendant une durée choisie, qui peut être par exemple de 1 heure à 3 jours. A la fin de cette période, la plaque est lavée à l'aide d'une solution adéquate qui permet l'élimination des cellules et la préservation des anticorps fixés à la plaque ainsi que des monokines produites pendant la culture. Un anticorps traceur, c'est-à-dire marqué par exemple par un enzyme ou un isotope, est ajouté dans les puits, ou bien ce traceur peut également être d'emblée présent dans le milieu de culture initial. La révélation du traceur et donc du médiateur à mesurer est alors directement réalisée dans la plaque de microtitration selon les méthodes conventionnelles.

Les traceurs, c'est-à-dire par exemple des anticorps marqués par un enzyme ou un isotope radioactif, peuvent être ajoutés dans les puits de culture soit au moment de la répartition initiale des cellules dans le milieu (le traceur pouvant être d'emblée incorporé à ce milieu), soit à tout autre moment plus tardif de la culture, voire après le lavage des cellules.

La quantité présente d'endotoxine peut être déterminée en mesurant la quantité de la dite substance induite, notamment de la monokine produite, par extrapolation par rapport à une courbe standard, réalisée à partir d'échantillons standard d'endotoxine cultivés dans les mêmes conditions, soit par une mesure directe de la radioactivité dans le cas d'un traceur radioactif, soit après réaction avec le révélateur enzymatique dans le cas d'un traceur enzymatique, soit par toute autre méthode conventionnelle.

Des cellules de toutes origines, dans leur milieu naturel ou non, peuvent être utilisées selon l'invention. On peut citer par exemple le sang complet dilué ou non, d'origine humaine ou animale, ou des cellules sanguines isolées et purifiées d'origine humaine ou animale ; des cellules isolées à partir d'un tissu solide de foie, peau, glandes, tumeurs, ou autres; des lignées cellulaires humaines ou animales.

Lorsque le test selon l'invention implique l'utilisation d'une lignée cellulaire qui peut être d'origine humaine ou animale, il peut s'agir, par exemple, de cellules isolées du sang humain ou animal telles que leucocytes, lymphocytes ou monocytes.

On peut, selon le procédé, augmenter la capacité des cellules de répondre aux endotoxines en augmentant l'expression du récepteur au LPS (CD14), soit par une présélection de cellules exprimant une grande densité de ce récepteur, soit en augmentant in situ son expression par des agents régulateurs de la production de la monokine à doser (de la l-25 OH vitamine D, de la LBP (LPS binding protein), du CD14 soluble, de l'acide rétinoïque, du facteur C5a du complément, de l'IFNgamma, ou une autre cytokine) qui peuvent être ajoutés au milieu réactionnel.

On peut avoir recours également, dans le procédé de l'invention, à des inhibiteurs, par exemple des anticorps neutralisant la liaison substances induites - récepteur ou substances induites - protéine de liaison, pour amplifier la détection de la substances induites.

Il importe que le milieu et les conditions de culture soient standardisés pour chaque application afin d'obtenir des résultats comparables et reproductibles. Dans la majorité des cas, on peut utiliser le RPMI 1640 comme milieu de culture ou de dilution de base et des incubations à 37° en atmosphère saturée en eau pour des périodes variant de 1 à 72 heures, plus généralement de 2 à 24 heures. De très nombreuses variantes sont évidemment possibles en ce qui concerne le milieu et les conditions de culture, telle que la durée, notamment selon le type cellulaire utilisé.

Le lavage est une étape importante du procédé selon l'invention, car il convient d'éliminer suffisamment les cellules qui adhèrent le cas échéant au support commun de la culture et du dosage, sans dégrader les éléments du dosage, afin d'éviter toute interférence avec les étapes finales du dosage telles que par exemple une réaction enzymatique ou une mesure d'absorbance. Les cellules qui adhèrent au fond des puits, par exemple, sont susceptibles d'activer le substrat de l'enzyme et de donner un faux signal positif. Le lavage doit permettre le détachement complet des cellules sans détériorer les complexes antigènes-anticorps.

Les "agents de détachement" cellulaires, qui sont de préférence ajoutés au milieu de lavage, comprennent notamment :
- des agents chelatant tels que EDTA, EGTA,
- des enzymes protéolytiques de type trypsine, dispase, ou autres,
- des détergents de type Tween, Triton, ou autres,
- des inhibiteurs d'adhésion, notamment héparines, ou peptidiques (par exemple, comprenant une séquence RGDS ou analogue),
- ou un mélange de ces différents facteurs.

Dans un mode préféré de réalisation de l'invention, les solutions de lavage mises au point contiennent des enzymes protéolytiques, par exemple de la Trypsine. Dans les conditions d'utilisation, ces enzymes protéolytiques ne détruisent pas les éléments du dosage (complexes Ag-Ac) fixés sur la phase solide.

Les conditions de lavage peuvent être manuelles ou, de préférence, automatiques et répétées de manière standardisée.

Selon l'invention, le dosage in situ est un dosage immunologiqe sur phase solide, la phase solide étant présente avec la culture cellulaire au moment de la production du paramètre à étudier.

Pour autant qu'il soit sur phase solide, le dosage peut obéir aux différents principes bien connus de l'homme de l'art, par exemple tests par compétition ou en sandwich. Les méthodes de révélation peuvent être de toutes natures connues de l'homme de l'art, enzymatiques, isotopiques, basées sur la luminescence, sur la fluorescence, sur la diffraction néphélométrique, etc...

Les méthodes de dosage qui peuvent être utilisées sont en particulier les :
- méthodes sandwiches de type ELISA utilisant des anticorps traceurs marqués à la péroxidase ou, bien entendu, avec d'autres enzymes que la péroxidase, ribonucléase, phosphate alcaline, acétylcholinestérase, et autres ;
- méthodes compétitives de type EIA utilisant des antigènes ou éventuellement des anticorps antiidiotypiques traceurs (cf. ELISA), pour autrant que l'anticorps de capture soit sur une phase solide de type tube/plaque/bille revêtue d'anticorps ;
- méthodes sandwiches de type IRMA utilisant des anticorps traceurs radioactifs (¹²⁵I, ¹³¹I, ³H,...) ;
- méthodes compétitives de type RIA utilisant des antigènes traceurs (cf. IRMA), pour autant que l'anticorps de capture soit sur une phase solide de type tube/plaque/bille revêtue d'anticorps ;
- toutes méthodes d'immunodosages de type sandwich ou compétition basées sur d'autres types de traceurs (luminescence, fluorescence...) et plus généralement
- toutes autres méthodes d'immunodosages sur phase solide basées sur d'autres principes que le sandwich ou la compétition, en particulier l'agglutination.

Les éléments de captures, fixés à la phase solide, peuvent être des anticorps, ou des récepteurs spécifiques de la substances induites à doser, ou tout autre ligand, notamment ligand spécifique de la substances induites à doser. Il en est de même des traceurs utilisés.

Les anticorps qui peuvent être utilisés peuvent être monoclonaux, oligoclonaux (c'est-à-dire qu'on utilise plusieurs anticorps monoclonaux reconnaissant des épitopes différents de la même substances induites) ou polyclonaux, entiers ou sous forme de fragments. Ils sont sélectionnés pour la qualité du dosage et sélectionnés ou non pour leurs propriétés biologiques concernant la production du paramètre à doser, sa stabilité, sa préservation, sa capture, son démasquage vis-à-vis des molécules liantes.

En effet, de préférence, les anticorps seront sélectionnés et choisis non seulement pour leurs qualités "statiques" dans le dosage mais aussi pour l'influence qu'ils peuvent exercer sur la production "dynamique" de la dite substance induite, notamment de la monokine (par exemple en interférant avec les boucles de régulation de la production), sur sa stabilité (par exemple en empêchant la dégradation de la dite substance induite), sur sa disponibilité (par exemple en empêchant la recaptation et consommation de la substance induite par des cellules ou en empêchant son masquage par des éléments solubles capables de la complexer), etc ...

Dans le cadre de certaines applications particulières, il peut être préférable, suivant l'effet recherché, que les anticorps participant au dosage interfèrent ou n'interfèrent pas avec les activités paracrines et autocrines des monokines dosées qui, le cas échéant, influencent les processus d'activation et de production de ces monokines. En effet, si c'était le cas, comme ces anticorps sont présents au moment de la stimulation, la production finale de certaines monokines pourrait être modifiée. Cela implique que les anticorps utilisés pour les dosages modifient ou ne modifient pas l'activité biologique des dites substances induites dosées (anticorps neutres), c'est-à-dire que ces anticorps doivent de préférence reconnaître ou non la dite substance induite par des épitopes distincts du site de liaison de la dite substance induite à son substrat (récepteur cellulaire).

Cette notion peut être illustrée par cet exemple :
L'IL1 produite par les monocytes potentialise l'activation des cellules T par la PHA. Les cellules T en retour produisent de l'IL4 qui inhibe la production d'IL1 par les monocytes. Si l'un des anticorps participant au dosage dynamique de l'IL1 bloquait son activité biologique sur les cellules T, la production d'IL4 serait diminuée et le rétrocontrôle négatif sur la production d'IL1 se ferait moins bien avec comme résultat une production accrue d'IL1 en présence d'endotoxines.

De façon non exhaustive, on peut citer les avantages suivants du procédé de l'invention :
1. Grâce au processus d'intégration culture/dosage, on aboutit à une très grande simplicité de réalisation et rapidité.
2. Le procédé ne nécessite pas le matériel de séparation cellulaire et cellules/surnageant indispensable selon les procédés conventionnels (par exemple centrifugeuses...).
3. Les manipulations des cellules sont très réduites d'où une réduction des causes d'activation ou de sélection involontaires des cellules fausant l'interprétation des résultats.
4. On observe une très grande reproductibilité des résultats.
5. Des possibilités d'automatisation de tout ou partie de la procédure sont beaucoup plus grandes, notamment suite à la suppression des étapes de récolte de surnageants, de stockage et de redistribution selon les procédés multiétapes conventionnels.
6. La production de la substance induite et les premières étapes du dosage sont réalisables dans l'environnement naturel (ou sélectionné) des cellules, enfin et surtout
7. On procure aux laboratoires de biologie clinique un accès plus facile et plus routinier à un nouveau test de pyrogénicité.

L'invention a également pour objet un format technologique ainsi que des "kits" standardisés poru réaliser un test de pyrogénicité impliquant la mise en oeuvre d'un procédé de dosage in situ.

Ces kits comprennent le(s) récipient-support(s) commun(s), selon l'invention, à l'étape de culture et d'initiation du dosage ainsi que éventuellement les milieux de culture, milieux de lavage, activateurs, révélateurs et échantillons standards du dosage, et autres réactifs nécessaires au dosage selon l'invention. Plus précisément, ces kits comportent :
- le dit récipient-support de culture celluliare servant de récipient de dosage, la dite phase solide sur laquelle sont fixés les éléments de capture de l'immunodosage, étant incorporée ou incorporable au dit récipient (c'est-à-dire physiquement dépendant ou indépendant du récipient) ;
- ainsi tous les éléments du dosage, y compris le traceur, des échantillons standard d'agents pyrogènes, ainsi que des milieux de culture, de dilution cellulaire, de lavage et des agents régulateurs de production des paramètres à mesurer le cas échéant.

Comme on l'a vu, le récipient-support solide sera de préférence sous forme de puits assemblables sous forme d'une plaque de titration ou sous forme de tubes qui pourront également être assemblés pour la commodité de l'utilisateur et l'adaptation aux instruments standardisés (par exemple laveur). D'autres supports pour des applications particulières (boîtes de pétri, lames de verre compartimentalisées, etc...) voire une dissociation physique entre le support de la culture et le support du dosage pour autant qu'ils soient destinés à être rassemblés lors de la production des paramètres à doser, par exemple des billes recouvertes d'Anticorps sont également appropriées.

Selon une variante, le traceur peut être incorporé sous forme séchée (par évaporation ou lyophilisation) dans les dits récipients, notamment dans des puits de la microplaque revêtus des anticorps participant au dosage. Dans ce cas particulier, il suffit d'ajouter la solution telle que le sang complet fraîchement prélevé dans les micropuits en présence d'un volume adéquat de milieu de culture et de l'échantillon d'agent pyrogène à doser.

Selon une variante, les échantillons standards peuvent être incorporés sous forme séchée (par évaporation ou lyophilisation) dans les dits récipients, notamment dans les puits de la microplaque revêtus des anticorps participant au dosage. Dans ce cas particulier, il suffit de réhydrater les standards avec de l'eau et d'ajouter le même volume de milieu de culture que pour l'échantillon.

D'autres caractéristiques et avantages de la présente invention apparaîtront à la lumière de la description détaillée qui va suivre.

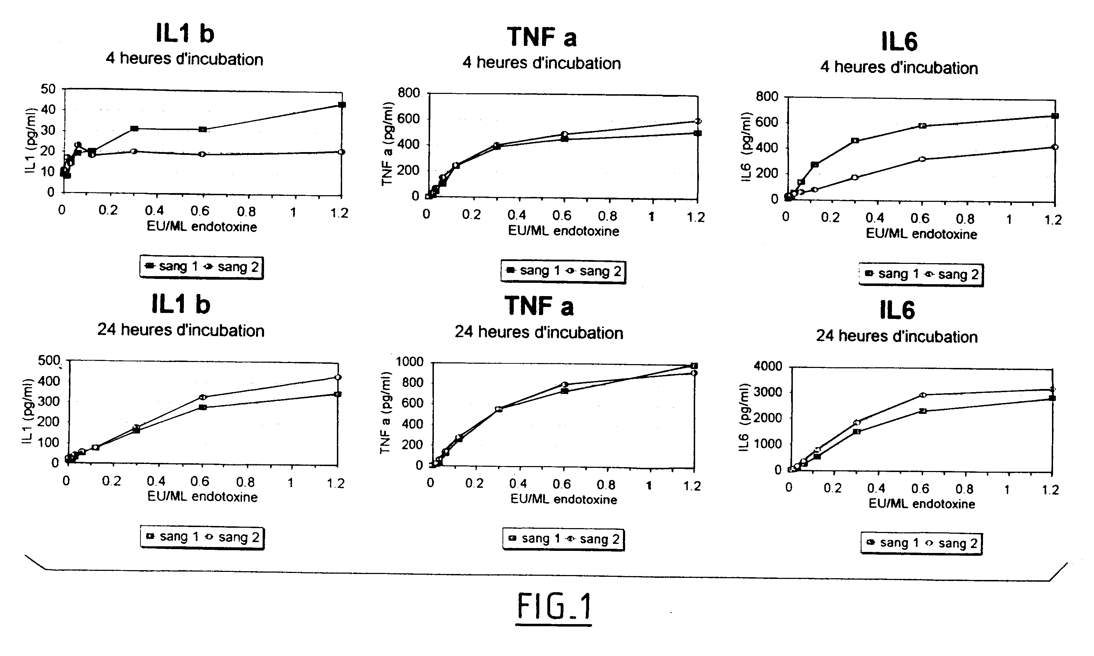

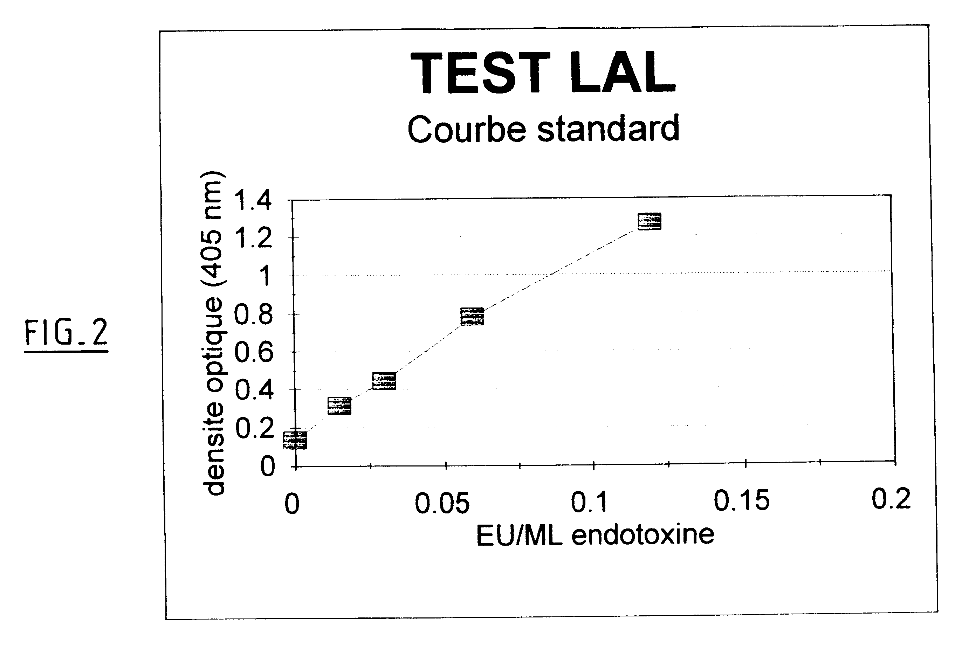

Des expériences ont été réalisées, basées sur la production de TNFalpha, IL1 bêta et IL6, dans des cultures de sang humain dilué 1/10 dans du milieu de culture (RPMI) après différents temps de culture en présence de doses croissantes de LPS, dont les résultats sont présentés dans les figures 1 et 2.

Le format technologique selon l'invention comporte par exemple le matériel suivant :
1. une lignée cellulaire (par exemple monocytaire, d'origine humaine ou animale) ne produisant pas spontanément de substances induites, par exemple de l'IL6, mais produisant la dite substances induites, par exemple de l'IL6, en présence d'endotoxine. On peut utiliser du sang complet d'origine humaine ou animale, ou encore des éléments cellulaires isolés (leucocytes, lymphocytes, monocytes, ...) du sang humain ou animal comme on l'a vu ;
2. une plaque de microtitration revêtue avec des anticorps anti-monokine, par exemple anti-I16 (dirigés contre de l'IL6 humaine ou animale en fonction de l'origine de la ligne cellulaire) ;
3. une fiole contenant un milieu de culture (par exemple RPMI 1640) pouvant facultativement contenir d'emblée un anticorps traceur spécifique anti-monokine, par exemple anti-Il6 (dirigés contre de l'IL6 humaine ou animale en fonction de l'origine de la lignée cellulaire) ;
4. une fiole contenant les solutions de lavage adaptées ;
5. une fiole de solution contenant des anticorps traceurs anti-monokine, par exemple anti Il6 (dirigés contre de l'IL6 humaine ou animale en fonction de l'origine de la lignée cellulaire), si ceux-ci ne sont pas incorporés dans le milieu ;
6. une fiole contenant le substrat de l'enzyme par exemple peroxydase avec lequel l'anticorps brutalement marqué;
7. une fiole de solution tampon pour diluer le substrat;
8. une fiole de solution d'arrêt, par exemple une fiole d'H₂SO₄ pour bloquer la réaction enzymatique.
9. un jeu de fioles contenant des solutions pyrogènes standard calibrées à base d'endotoxine LPS.

Le test peut se dérouler comme suit.
L'échantillon à tester ou les solutions pyrogènes standards sont dilués dans le milieu de culture et incubés dans les puits revêtus avec les anticorps de capture anti-monokine et, facultativement, en présence de l'anticorps traceur anti-monokine et en présence d'un nombre déterminé de cellules de la lignée (ou du sang complet ou des cellules isolées) pendant un temps déterminé. Après la période d'incubation, la plaque est lavée à l'aide des solutions de lavage adéquates et l'anticorps traceur est ajouté dans les puits, si celui-ci n'est pas d'emblée présent dans le milieu de culture initial.

La révélation du traceur et, par conséquent, de la substances induites produite, est alors réalisée dans la plaque de microtitration selon les méthodes conventionnelles.

Alternativement, la culture des cellules en présence de l'échantillon à tester ou des solutions pyrogènes standards peut être réalisée séparément après quoi les surnageants sont récupérés et leur contenu en substances induites mesuré séparément par les méthodes de dosage conventionnelles.

Si l'échantillon testé comprend des agents pyrogènes (LPS), les cellules vont être activées et vont produire de la substances induites proportionnellement à la quantité de pyrogène présent dans l'échantillon. Cette quantité de pyrogène pourra être quantifiée par référence à la substances induites induite par les solutions de pyrogènes standard.

### PROCEDURE :

- on délimite la partie de la plaque qui va servir aux standards et aux échantillons à tester;
- on dilue l'anticorps traceur concentré à sa concentration de travail dans le volume requis de milieu de culture;
- on remplit tous les puits avec 200 µl de la solution milieu de culture-traceur;
- pour la gamme standard, on ajoute 25 µl des différents points standards d'endotoxine dans les puits correspondants;
- pour les échantillons, on ajoute 25 µl des différents échantillons à tester dans les puits correspondants;
- dans tous les puits, on ajoute 25 µl d'une solution contenant un nombre déterminé de cellules de la lignée monocytaire;
- on incube pendant un temps déterminé (par exemple 4 heures) à 37 degrés centigrades;
- on lave la plaque avec les solutions de lavage fournies;
- on ajoute 200 µl par puits de la solution substrat diluée dans le tampon du substrat;
- on incube 15 minutes sous agitation à température ambiante;
- on ajoute 50 µl de la solution bloquante d'H₂SO₄;
- on lit la plaque dans un spectrophotomètre et on extrapole la concentration d'endotoxine présente dans les échantillons par rapport à la courbe standard.

Les dosages en IL1bêta, IL6 et TNFalpha ont été réalisés avec les kits de dosage IL1bêta EASIA^{R}, IL6 EASIA^{R} et TNFalpha EASIA^{R} commercialisés par la société MEDGENIX DIAGNOSTICS.

### RESULTATS:

Ils sont présentés sur les figures.

### Figure 1 :

La figure 1 montre la production d'IL1, d'IL6 et de TNFalpha dans des cultures de sang humain dilué 1/10 dans du milieu de culture (RPMI) après différents temps de culture en présence de doses croissantes d'ENDOTOXINES. L'endotoxine utilisée est le standard endotoxine E. Coli 0111:B4 LPS utilisé comme standard dans le kit commercial de dosage des endotoxines: "Coatest Endotoxine" commercialisé par la société CHROMO-GENIX (Taljegârdsgatan 3, S-461 53 Mölndal, Suède). Ce standard est lui-même, suivant les indications du fabricant, calibré sur le standard SDA EC-5 lot F et le standard WHO 84/650.

Les graphiques IL1bêta 4 heures/24 heures d'incubation montrent la production d'IL1 après 4 et 24 heures de culture du sang complet de deux donneurs sains en présence de doses croissantes d'endotoxines.

Les graphiques IL6 4 heures/24 heures d'incubation montrent la production d'IL6 après 4 et 24 heures de culture du sang complet de deux donneurs sains en présence de doses croissantes d'endotoxines.

Les graphiques TNFalpha 4 heures/24 heures d'incubation montrent la production de TNTalpha après 4 et 24 heures de culture du sang complet de deux donneurs sains en présence de doses croissantes d'endotoxines.

### Figure 2 :

La figure 2 montre, à titre de comparaison, une courbe standard obtenue avec le même standard que celui utilisé pour les expériences de la figure 1, suivant le protocole du test LAL (end-point + microplate method) en conformité avec les instructions du fabricant. Les courbes standard selon l'invention présentées à la figure 2 montrent une réponse aussi sensible.

### CONCLUSIONS

Le TNFalpha et l'IL6 montrent une réponse très sensible au standard endotoxine chez les deux donneurs après 4 heures de culture.

L'IL1B montre une réponse sensible au standard endotoxine chez les deux donneurs après 24 heures de culture mais pas de réponse significative après seulement 4 heures d'incubation.

## Revendications

1. Test de pyrogénicité consistant dans la détection d'agent(s) pyrogène(s) tel(s) que les endotoxines, en particulier les lipopolysaccharides (LPS), dans une solution à usage médical ou biologique, dans lequel :
1. On mesure la quantité de substance induite telle que des monokines, des prostaglandines, des protéases cytolitiques, l'alpha2-macroglobuline les ROI (reactive oxygen intermediates) dans la dite solution avant et après culture dans la dite solution de cellules ne produisant des dites substances induites qu'en présence du(des) dit(s) agent(s) pyrogène(s) ou de cellules dont la production de la dite subtance induite est accrue en présence du(des) dit(s) agent(s) pyrogène(s).
2. On évalue la quantité d'agents pyrogènes dans la dite solution par rapport à la différence de quantité de la dite substance induite après et avant la dite culture à l'aide d'une courbe de corrélation entre la quantité d'agent(s) pyrogène(s) et la quantité de la dite substance induite, courbe réalisée en mesurant, dans les mêmes conditions, la quantité de la dite substance induite produite dans des échantillons de solutions initialement dépourvues de la dite substance induite, calibrées en agent(s) pyrogène(s) et cultivées dans les mêmes conditions.

2. Test selon la revendication 1, dans lequel on effectue la culture cellulaire, et l'on évalue la quantité de la dite substance induite produite par dosage immunologique sur phase solide in vitro, caractérisé en ce que :
a) la culture cellulaire est effectuée, éventuellement en présence du traceur, dans un récipient qui incorpore la phase solide du dosage sur laquelle sont fixés les éléments de capture spécifiques de la substance induite à doser, et en ce que
b) lorsque le temps de culture est écoulé, après lavage de la phase solide et incubations des éventuels différents autres réactifs nécessaires au dosage, notamment un substrat du traceur, en présence de la phase solide, on lit le signal émis par le traceur fixé à la phase solide ou par son dit substrat.

3. Test selon la revendication 2, caractérisé en ce que dans l'étape b), le traceur et la phase solide sont incubés dans le récipient ayant servi à la culture.

4. Test selon l'une des revendications 2 ou 3, caractérisé en ce que le signal émis est lu dans le récipient ayant servi à la culture.

5. Test selon l'une des revendications 2 à 4, caractérisé en ce que la phase solide consiste en la surface intérieure du récipient de culture sur laquelle sont fixés les éléments de capture spécifiques de la substance induite à doser.

6. Test selon l'une des revendications 2 à 4, caractérisé en ce que la phase solide est constituée par un ou des élément(s) physiquement indépendants du récipient de culture cellulaire.

7. Test selon la revendication 6, caractérisé en ce que la phase solide est consituée par des billes, un élément solidaire d'une pièce de préhension, revêtues desdits éléments de capture spécifiques de la substance induite à doser.

8. Test selon l'une des revendications 2 à 7 dans lequel le dosage immunologique est de type compétition sur phase solide, ou de type sandwich.

9. Test selon l'une des revendications 1 à 8 dans lequel l'élément de capture est constitué par des anticorps monoclonaux ou polyclonaux.

10. Test selon l'une des revendications 2 à 10 dans lequel le dosage est révélé par une méthode enzymatique, isotopique, par luminescence, par fluorescence, ou par diffraction néphélométrique.

11. Test selon l'une des revendications 2 à 10 dans lequel le composant traceur du dosage est présent dès l'initiation de la culture sur le support ou ajouté ultérieurement.

12. Test selon l'une des revendications 2 à 11 dans lequel la substance induite est une monokine telle que le TNFalpha, l'IL₁, l'IL₆, ou l'IL₈.

13. Test selon l'une des revendications 2 à 12 dans lequel l'agent pyrogène est un lipopolysaccharide (LPS), notamment provenant de la paroi de bactérie gram⁻.

14. Test selon l'une des revendications 2 à 13, caractérisé en ce que les anticorps participant au dosage à titre de traceur ou d'élément de capture de la molécule à doser fixés à la phase solide, reconnaisent la monokine par des épitopes n'affectant pas la production de la dite monokine et/ou son activité biologique.

15. Test selon les revendications 2 à 14, caractérisé en ce que, lors de la culture, on ajoute au milieu des agents régulateurs de la production de la dite substance induite à doser.

16. Test selon la revendication 15, caractérisé en ce que l'agent régulateur de la production de la monokine à doser est de la l-25 OH Vitamine D, de la LBP (LPS binding protein), du CD-14 soluble, de l'acide rétinoïque, du facteur C5a du complément, de l'IFNgamma, ou une autre cytokine.

17. Test selon l'une des revendications 1 à 16 caractérisé en ce que la dite solution à tester est une solution injectable dans un mammifère y compris l'homme, ou une solution de milieux de culture de cellules pour usage d'analyse biologique.

18. Test selon l'une des revendications 1 à 17 caractérisé en ce que la lignée cellulaire cultivée est une lignée d'origine humaine ou animale.

19. Test selon l'une des revendications 1 à 18 dans lequel les cellules sont représentées par du sang complet d'origine humaine ou animale, éventuellement dilué, ou des éléments cellulaires isolés du sang humain ou animal tels que leucocytes, lymphocytes, monocytes.

20. Test selon l'une des revendications 2 à 19 caractérisé en ce que la solution de lavage de la phase solide avant lecture du signal est une solution d'enzyme protéolytique notamment de trypsine.

21. Format technologique ou kit utile dans le test de pyrogénicité selon l'une des revendications 2 à 20, caractérisé en ce qu'il comporte le dit récipient-support de culture cellulaire servant aussi de récipient d'immuno-captation, la dite phase solide sur laquelle sont fixés les éléments de capture de l'immunodosage étant incorporée ou incorporable au dit récipient.

22. Format selon la revendication 21, caractérisé en ce que la phase solide est constituée par la surface intérieure du récipient-support de culture.

23. Format technologique selon la revendication 22, dans lequel le récipient-support solide est constitué par des puits, éventuellement amovibles, d'une plaque de titration, par des tubes, ou par tout autre récipient de culture cellulaire approprié au dosage immunologique.

24. Format technologique selon la revendication 23, dans lequel le récipient de culture cellulaire et d'immuno-captation est physiquement indépendant de la phase solide du dosage.

25. Format technologique selon l'une des revendications 21 à 24, dans lequel sont fournis tous les éléments du dosage, y compris des échantillons standards d'agent(s) pyrogène(s), le traceur, son éventuel substrat ainsi que des milieux de culture, de dilution cellulaire, de lavage et des agents régulateurs de la production de substance induite à mesurer le cas échéant.

26. Format selon l'une des revendications 21 à 25, caractérisé en ce qu'il comprend un traceur présent sous forme séchée dans les puits de la microplaque.

27. Format selon l'une des revendications 21 à 26, caractérisé en ce qu'il comprend des échantillons standard d'agents pyrogènes présents sous forme séchée dans les dits récipients, notamment dans les puits de la microplaque.
